# EUROPEAN PATENT APPLICATION

(11) **EP 0 967 288 A1**
(43) Date of publication of application: **29.12.1999**
(21) Application number: 98111001.8
(22) Date of filing: 16.06.1998
(51) Int. Cl.: C12N 15/87, A61K 48/00, A61K 38/17

(54) **Transfection system for the transfer of nucleic acids into cells**

(71) Applicant: Hoechst Marion Roussel Deutschland GmbH, 65929 Frankfurt am Main (DE)
(72) Inventor: Chandra, Prakash, Prof. Dr., 60489 Frankfurt (DE); Chandra, Angelika, Dr., 60489 Frankfurt (DE); Demirhan, Ilhan, Dr., 65931 Frankfurt (DE); Hasselmayer, Oliver, Dl., 63065 Offenbach (DE)

(57) **Abstract**

This invention relates to a transfection system comprising histones or derivatives thereof and nucleic acids that should be brought into cells, the use of the new transfection system for any kind of nucleic acid transfection, especially the use of the transfection system for gene therapy and for the preparation of pharmaceuticals.

## Description

The invention relates to a new transfection system comprising a histone or a derivative thereof and a nucleic acid, the use of the new transfection system for any kind of nucleic acid transfection, especially the use of the new transfection system for gene therapy and for the preparation of pharmaceuticals.

One of the major problems when foreign genes should be introduced and expressed in eukaryotic cells is that the polynucleotides must cross several membranous barriers to reach and enter the cell nucleus. The first barrier, the cytoplasmic membrane, can be traversed by many different methods (for reviews, see Keown et al., 1990) like calcium phosphate precipitation (Loyter et al., 1982; Chen et al., 1988), electroporation (Neumann et al., 1982; Othani et al., 1989), liposome packaging of DNA (Fraley et al., 1982; Nicolau et al. 1987, Mannino et al., 1988), with (Wang et al. 1987; Machy et al. 1988; Kanedi et al., 1989) or without specific targeting of the liposomes, lipofection (Felgner et al., 1987; Holt et al., 1990) and microinjection (Graessmann et al.). Also described is the use of polycations such as Polybrene (Kawai et al., 1984), DEAE-dextran (Chandra et al., 1988; Demirhan et al., 1994; Graham et al., 1973; McCutchan et al., 1968; Choi et al., 1988) or the use of proteins, which are taken up by the process of receptor-mediated endocytosis (Wagner et al., 1990; Zenke et al., 1990; Wu et al., 1989; Wu et al., 1991). The method of receptor-mediated gene transfer is a specific approach, but it is limited by the presence and the number of receptors, which can be modulated in certain cases (Cotten et al., 1990; Marceau et al., 1990; Curiel et al., 1992).

Furthermore, there are other several drawbacks that limit foreign gene delivery and expression in target cells. Such limitations include insufficient condensation of plasmid DNA and the lack of specific mechanisms for DNA to escape from endosomes, and to enter the cell nucleus. There are described some ways to improve these methods and to compensate the above mentioned drawbacks like an increase in reporter gene expression when plasmid DNA is compactly packed into toroid structure by protein carriers (Wagner et al., 1991), which is dependent upon having the right combination of the length and the amount of the DNA-binding part in the carrier complex. To increase the escape of DNA from endosomes, some reports describe to the use of the property of some viruses to destroy endosomes (Curiel et al., 1991; Wagner et al., 1992a; Wagner et al., 1992b; Cotten et al., 1992; Cotten et al., 1993; Plank et al., 1994; Wu et al., 1994; Zauner et al., 1995).

Histones may for example be used to condensate DNA as described in WO 96/20732 (p. 13, I. 21-32). WO 96/20732 further describes the aspects which must be fulfilled by such a DNA condensate in order to speak of efficient transfection. However, subsequently WO 96/20732 describes not the use of histones for transfection or transfection experiments carried out by using histone proteins.

Bielinska A.U. et al. (Biochim. Biol. Acta1353, 180-190, (1997)) investigated the effect of various cationic proteins on transcription by means of a cell-free transcription-translation assay (Materials and Methods, p. 181-183) and the structure of a DNA-histone tetramer by a gel shift assay. It was found that the DNA-histone complex has, by contrast with a DNA-dendrimer complex, no transcription activity (abstract; p. 180,1. 12-13).

Dubes G.G. et al. (Protoplasma 96, 209-223, (1978)) report a sensitizing effect of cells by basic proteins such as protamine and histone. Cell "sensitization" is performed by incubating the cells solely with the basic proteins (1µg/ml); then the cells are washed three times (p. 210-212). Cells were then transfected with poliovirus RNA or polioviruses, and the infectivity was quantified by means of the plaques. It was found that the transfection rate was 10⁴ - 10⁵ higher with "sensitized cells". No incubation of histone with DNA or the use of histones in the transfection method itself is described.

Other transfection experiments are performed in the presence of histone proteins, but the histones are not used as DNA-carriers, they are only used as enhancers, additionals, cofactors or auxiliarys in combination with a known transfection method (variation of a known transfection method) or in addition to a known transfection reagent, e.g. as enhancer in the CaCl₂ method or as cofactor in receptor-mediated transfection. An enhancing effect of a particular histone type is found in some experiments, but the result are inconsistend with respect to a particular histone type.

Such an enhancer effect for histones is for example described in DE 196 16 645 A1. In DE 196 16 645 A1 an isolation method for nuclear proteins is described and the transfection activity of theses proteins including the histones H3 and H4 is investigated. The transfection method used is a modification of the calcium phosphate/DNA coprecipitation method, since it is disclosed that calcium must be added both to the complex formation buffer (col. 5, I. 19-22) and to the transfection medium (col. 3, I. 49-56; col. 5, I. 30-33).

The transfection method described in WO 96/14424 is also a variation of the calcium phosphate/DNA coprecipitation method which is used as the initial method (p. 3, I. 15-21). The variation comprises adding the histones (especially H2A) to calcium phosphate/DNA precipitates which have already formed (p. 2, I. 25-27; p. 3, I. 30-33, table 1; p. 15, claim 9). With this method an enhancing effect of histones on the transfection efficiency is observed (p. 2, I. 36 to p. 3,I. 1). Ca²⁺ in the form of calcium chloride or calcium nitrate is also an essential constituent of the described transfection kit (p. 3,I. 2-9; p. 15, claim 8). Moreover, WO 96/14424 teaches that histones alone lead to no transfection, i.e. histones are inactive as transfection reagent (p. 4, I. 5-7).

DD 256 148 A1 in addition describes a variation of the well-known Ca²⁺ transfection method by using calcium phosphate and as auxiliary component histone proteins. DD 256 148 A1 futhermore confirms the teaching of WO 96/14424 that histones are unable to transfect cells in the absence of calcium phosphate (p. 6, example 2, I. 6-10). In addition, it is described that histones stimulate transfection only if HMG-1 proteins are present.

Chen et al. (Human Gene Therapy 5 (4), 429-435, (1994)) describe a transfection method which is both, a variation of the calcium phosphate/DNA coprecipitation method and a variation of the receptor-mediated transfection method using the asialoglycoprotein (ASGP) receptor (p. 430, left-hand col., I. 1-3). The transfection is carried out in the presence of calcium chloride (final concentration: 5 mM (p. 431, left-hand col.)). In addition, the authors report that the histones have to be galactosylated (p. 430, right-hand col.), whereas non-galactosylated histones are unable to ensure DNA transfer (p. 433, left-hand col., I, 1-2).

In DE 44 18 965 A1 a receptor-mediated transfection method in which the transferrin/transferrin receptor system or the streptavidin system is used as transfection system is described. The histones mentioned in col. 2 (I. 18-44) are used merely as additional or auxiliary component in the described transfection system.

In the study performed by Wagner E. et al. (Proc. Natl. Acad. Sci. 88, 4255-4259, (1991)) different types of histones (H1, H3 and H4) and other cationic proteins were tested as cofactors in a receptor-mediated transfection system. The transfection system consists of the DNA to be transferred and a transferrin-polylysine conjugate (p. 4255, left-hand col., 2nd paragraph, section (ii); "Materials and Methods", right-hand col., section "Cell culture and transfection"). The complex formation of the transferrin-polylysine conjugate is a precondition for the stimulating effect of histones and other cationic proteins (p. 4257, left-hand col., 4th paragraph, last 5 lines).

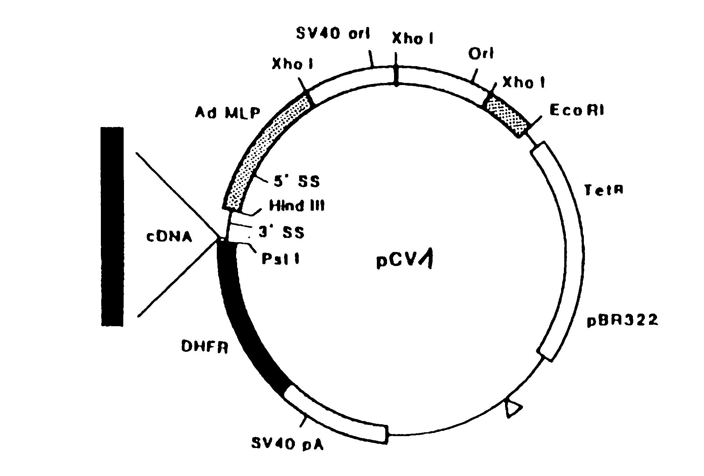

Obaru K. et al. (Human Gene Therapy 7, 2203-2208, (1996)) used the DEAE-dextran method for transfection (abstract, p. 2203, I. 6-7; p. 2204-2205 "transient expression assay") of an expression vector which comprises the LTR sequence of HIV-I and a sequence that encodes tat protein (p. 2204, Figure 1).

In Singh D. et al. (Nucleic Acids Research 24 (15), 3113-3114, (1996)) a retrovirus mediated transfection method is used and the potential of various substances, including histones, for increasing the efficiency of retrovirus mediated transfection is described. An enhancing effect of the histone H2A enhanced retrovirus mediated transfection is observed (p. 3113, right-hand col., 2nd paragraph and p. 3114, Figure 1).

Felgner P.L. (Adv. Drug Delivery Rev. 5, 163-187, (1990)) reviews the current transfection methods and possible ways of increasing their efficiency. For example, the use of cationic lipids for increasing transfection efficiency is proposed. In this connection an enhancing effect of histones and protamines is diclosed (p. 166, 2nd paragraph, I, 2-4).

In WO 95/34647 the use of "synthetic intracellular localization peptides" (p. 5, I. 26-31; p. 22-25) as enhancers (e.g. abstract) for conventional gene transfer systems is disclosed and in all transfection experiments described in the application the lipofection reagent Lipofectin is used (p. 17, I. 19-20; p. 21, I. 6). This is also the case in the experiment were histone protein is used as enhancer (p. 19; table 5).

Fritz et al. (Human Gene Therapy 7, 1395-1404 (1996)) performed experiments werein DNA-binding histone fractions were added to cocktails in which cationic liposomes (Lipofectin or Lipofectamine) were present in order to enhance transfection efficiency (e.g. p. 1402, 2nd col.). In the study a recombinant histone fusion protein (NLS-H1) which comprises the carboxy-terminal end of histone H1 (AA 99-108) and the N-terminal end of SV40 T antigen (p. 1396; I. 4-7) is used. Further, this study discloses, that H3 and H4 have no effect on transfection efficency (p. 1402, I. 4-7).

Similar investigations and result are described in Hagstrom J.E. et al. (Biochim. Biophys. Acta 1284, 47-55 (1996)). They used a transfection system which transfection comprises the DNA to be transfected (in the form of a plasmid), anionic liposomes and cationic DNA-binding protein (abstract (p. 47); introduction (p. 47, right-hand col., I, 3, 4 in the 2nd paragraph); (p. 48, left-hand col., 1st paragraph)). The cationic DNA-binding protein consists of a construct which comprises the nuclear localization sequence of the SV40 T antigen and of histone H1.

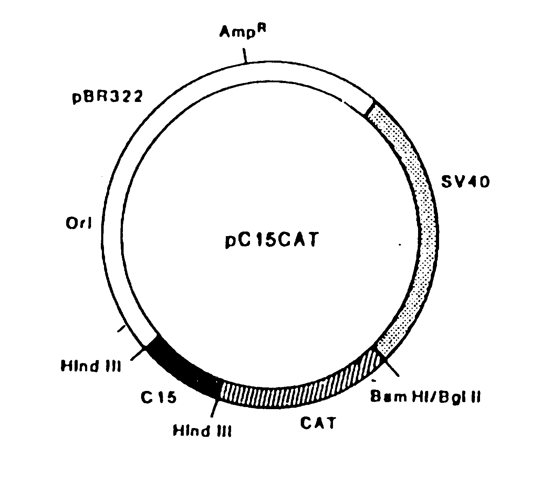

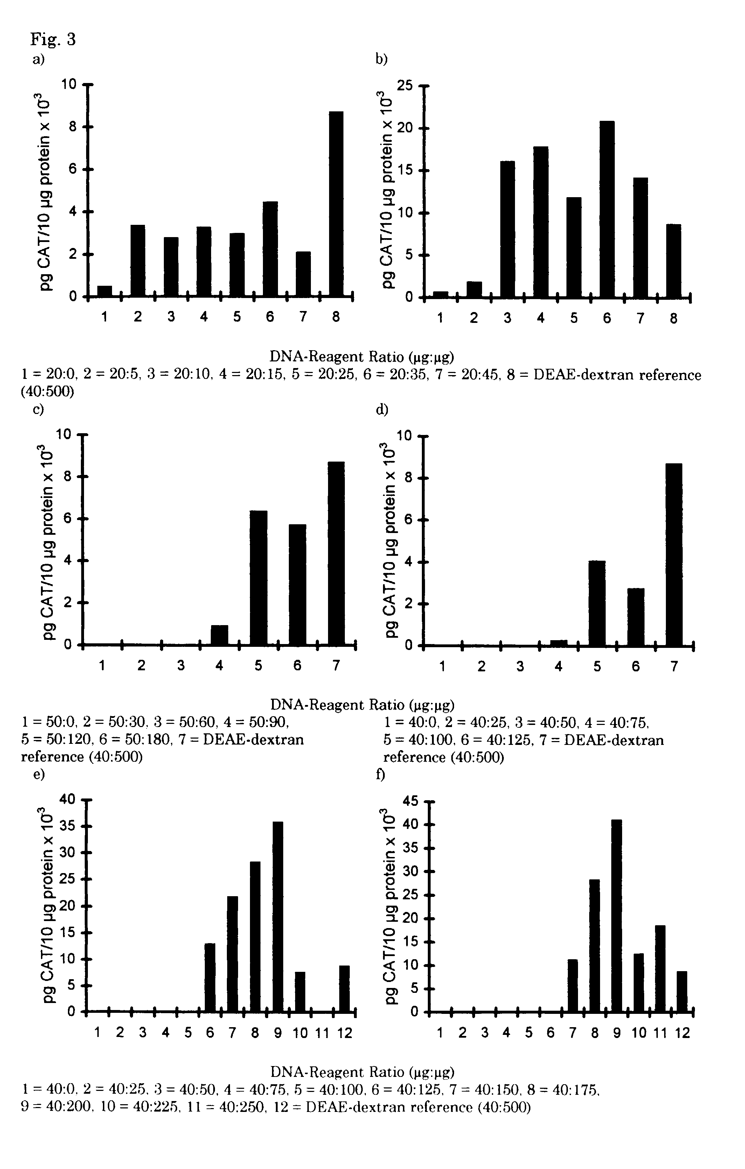

Boulikas, T. et al. (Int. J. of Oncology 10, 317-322, (1997)) compared three transfection methods all of which are based on the use of liposomes (p. 319, Fig. 2) and thus comprises a liposomal transfection strategy. Histones were used as enhancer in addition to protamines, polylysine and poly(E:K) in the transfection systems (p. 319, Fig. 3; p. 318, left-hand col., last paragraph; p. 319, left-hand col., 2nd paragraph).

Balicki D. et al. (Mol. Med. 3, 782-787, (1997)) used in their transfection experiments polylysine, polyarginine, and their combination with histones, also in combination with phospholipids. They found significant transfection only with the histone H2A (p. 784, left-hand col., 3rd paragraph; p. 784, Figure 1). Furthermore, it is teached that the transfection efficiency is a function of the tris acetate buffer concentration and thus the tris acetate buffer concentration has a crucial significance on transfection efficency (p. 785, left-hand col., 1st paragraph; p. 785, Figure 3).

Thus, the teachings with respect to in the activity of a particular histone type to modulate the transfection efficiency of a particular transfection method are not consistent. Furthermore, there is no teaching to use a histone protein as transfecting reagent. On the contrary, several investigators found that histone proteins alone lead to no transfection (Fritz et a. (1996); WO 96/14424; DD 256 148 A1).

The present invention aims to provide a universal transfection system that can be used to bring a nucleic acid into a prokaryotic and/or an eukaryotic cell, in particular into the nuclei of an eukaryotic target cell in a simple and effective manner.

According to the invention the problem is solved by providing a transfection system that comprises 1. a basic protein that tends to aggregate with a nucleic acid and which has a nuclear localization signal (NLS) responsible for targeting the protein into the nucleus and 2. a nucleic acid and 3. optionally additional compounds.

Transfection means that a nucleic acid, such as for example DNA is brought into a cell, in particular transfection means that a nucleic acid is brought into an eukaryotic cell and that the nucleic acid brought into a cell is functionally active.

Transfection system means a system that comprises the nucleic acid that should be transferred/transported. The transfection system comprises a transfecting reagent that can be used to transfer the nucleic acid.

The transfecting reagent is preferably a basic protein that tends to aggregate with the nucleic acid to be transferred and which has a nuclear localization signal (NLS) responsible for targeting the protein into the nucleus.
The transfecting reagent is preferably a histone protein or a derivative thereof, most preferably a histone H3 and/or a histone H4 or a derivative thereof.

One of the most favorable embodiments of the invention relates to a transfection system that comprises 1. a histone protein or a derivative thereof and 2. a nucleic acid and 3. optionally additonal compounds.

Preferably the transfection system according to the invention contains non of the known transfection reagents, such as for example high Ca²⁺ concentrations (like in the CaCl₂ method), liposomes, lipofectins, receptors and/or DEAE-dextran in a concentration known to be used in these transfection methods.

Histones are basic proteins which are components of the chromatin structures naturally occuring in most of the nuclei of eukaryotic cells. Histones are characterized by having an unusually high amount of basic amino acids (about 20 to 30 %) and a relative molecular weight of about 11.000 to 24.000 Da (e.g. Benjamin Lewin (1983) "Genes III", John Wiley & Sons, Inc. New York, p. 519-558).

Five different classes of histones are known: The histones 2A, 2B, 3 and 4 (H2A, H2B, H3, H4) form a histone octamer which interacts directly with DNA to form the first level of particles in the chromatin , whereas the histone 1 (H1) is concerned with interactions between these particles.

The histones H3 and H4 are the most Arginine-rich histones. For example, bovine histone H3 contains about 13 % Arginin, 10 % Lysin and has a relative molecular weight of about 15,3 kDa. The bovine histone H4 contains about 14 % Arginin, 11 % Lysin and has a relative molecular weight of about 11,2 to 11,3 kDa.

The histones H3 and H4 are among the most conserved proteins known in evolution. For example the sequences of the 102 amino acid sequence of bovine H4 differs only in two amino acids from that of the pea and the sequences of the 135 amino acid sequence of bovine H3 differs in 4 amino acids from that of the pea (Lexikon der Biochemie und Molekularbiologie (1991) Herder Verlag, Freiburg).

Histone 3 and histone 4 are small positively charged proteins which can bind nucleic acids by ionic interactions. In addition H3 and H4 have nuclear location signal (NLS) sequences. This property leads to rapid accumulation of H3 and H4 in the nucleus both by diffusion trough and active transport into the nucleus (Bonner, 1975; Dingwall *et al.,* 1984; Moreland et al., 1987, Chen et al. 1994).

In a preferred embodiment of the invention, the transfection system comprises histone 3 (H3) and/or a derivative thereof and/or histone 4 (H4) and/or a derivative thereof. In another preferred embodiment the invention comprises a mixture of histones or derivatives thereof preferably of H3 and H4 and optionally other compounds.

Derivatives of histones comprise any histones, in particular naturally occuring histones that have been modified or altered in any way with the provise that their specific ability to aggregate with nucleic acids and translocate them into the nuclei of cells is maintained. Modifications are for example deletion and/or addition of one or more amino acids from/to the aminoterminal and/or from/to the carboxyterminal end of the histone protein and/or from/to somewhere in the middle of the amino acid sequence. Further examples for alterations are amino acid changes or amino acid substitutions respectively within the naturally occuring amino acid sequence of a histone and/or modifications of amino acids, e.g. acetylation (e.g. E-aminogroup of lysine residues), ADP-ribolysation (e.g. glutamine residues), methylation and/or phosphorylation (e.g. hydroxy groups of serine and/or threonine residues).

The invention also comprises a method of making the transfection system. The transfection system can be made by mixing a histone with a nucleic acid. Upon mixing the histone may be incubated with the nucleic acid. In a special embodiment the incubation time is about 5 to 60 minutes, preferably 10 to 30 minutes, most preferably about 15 minutes.

For transfection and/or in the transfection system and/or for the method of making the transfection system respectively e.g. a histone protein that has been isolated and/or a recombinant histone protein ("histone protein" and "histone" are used in the same sense) can be used.

The histone can for example be obtained by isolation from any kind of cells and/or cell lines, any kind of animal tissue, preferably thymus or from human/animal umbilical cords, human/animal transplantation waste product, human/animal biopsy-material, human/animal tumor tissue (e.g. material which has been removed by surgery) and/or human/animal placenta. A method for the isolation of histone protein from tissue and/or cells is for example described in E.W. Johns, Biochem. J. 92, 55-59 (1964).

A recombinant histone protein can be obtained by expression of histone-encoding DNA, e.g. a gene in prokaryotic and/or eukaryotic cells. For example human histone protein can be obtained by cloning a DNA that encodes human H3 (for example a DNA encoding for the amino acid sequence in table 7) and/or a DNA that encodes human H4 (for example a DNA encoding for the amino acid sequence in table 5) in an appropiate (expression-) vector. Such expression vector can than be used to express the histone in a suitable host cell. Histones can also be obtained from transgenic animals which (over)-express a particular histone-transgene. Such histone-transgene would e.g. comprise a histone encoding DNA sequence under the control of a suitable promoter.

The histone-encoding DNA can e.g. be a gene or a cDNA or a part thereof. For example, the histone-encoding DNA may be a cDNA that encodes for a protein with the amino acid sequence SEQ ID NO.: 1, SEQ ID NO.: 2, SEQ ID NO.: 3 or SEQ ID NO.: 4.

Sequences of histone encoding DNA can be obtained from genetic sequence databases such as for example from the NCBI (National Center for Biotechnology Information), EMBL (European Molecular Biology Laboratory) and/or the Internet for example under
http://www.ncbi.nlm.nih.gov.Baxevani/HISTONES/table.html.

Some histones are also commercially available, e.g. H3 and H4 (isolated from calf thymus) and can be obtained from e.g. Fluka Chemie AG, Neu-Ulm (No. 53411, Histone H3, Johns Fraction f3; No. 53413, Histone H4, Johns Fractione f2a). The histones from Fluka are prepared by a modified preparation process according to E.W. Johns, Biochem. J. 92, 55-59 (1964).

The histone protein can be from any kind of animal and/or eukaryotic microorganism and/or plant , e.g. from a vertebrate and/or a mammalian such as for example rodent, primate, sheep, goat, dog, cow, pig, bird and/or amphibian, reptile. This is of particular interest, since for example H3 and H4 are highly conserved between different species. The consensus sequence for H3 is given in table 6. The amino acid sequence of human H3 (table7) differs only in 6 positons (5 within the sequence, 1 at the carboxy terminal end and 1 insertion at the carboxy terminal end) from the consensus sequence. The consensus sequence for H4 is given in table 4. The amino acid sequence of human H4 (table 5) differs only in 3 amino acids from the consensus sequence.

The nucleic acid which should be transferred can for example be a DNA, RNA, oligonucleotide or derivative thereof. A derivative is for example a nucleic acid in which the phosphodiester internucleoside linkage, the sugar unit, the nucleobase and/or the sugar-phosphate backbone has been modified and/or replaced. Derivatives include also nucleic acid salts, in particular a physiological tolerated salt of a nucleic acid.

The nucleic acid can for example be isolated and/or synthesized by chemical and/or enzymatical methods. The nucleic acid may have any kind of length. For example the nucleic acid may be relatively short, for example it may comprise only 10 or less, 20, 30, 40, 50 or 100 nucleotides. On the other hand the nucleic acid may comprise 1000, 10000, 100000, 1000000 or more nucleotides. The nucleic acid may be a chromosome.

The nucleic acid may comprise for example any kind of gene, derivatives and/or parts thereof, any kind of cDNA, derivatives and/or parts thereof. The nucleic acid can be linear and/or circular (e.g. plasmid, cosmid, artificial chromosome).

The nucleic acid may contain a sequence that encodes for a protein, e.g. a soluble and/or unsoluble and/or membrane bound protein. The nucleic acid may contain a sequence that encodes for example for a peptide, enzyme, receptor, cell-adhesion protein, immunomodulator, growth factor, tumorsupressor, regulator protein as e.g. transcriptionfactor, co-factor and/or trans-activating factor.

In a special embodiment of the invention the nucleic acid contains a sequence that encodes for a protein or a peptide which is involved in the formation or the development of a disease.

In another embodiment of the invention the nucleic acid may function, in particular if the nucleic acid is a RNA or contains a sequence that encodes a RNA and/or the nucleic acid is an oligonucleotide or a modified derivative thereof as antisense oligonucleotide, as triplex-forming oligonucleotide and/or as ribozyme (e.g hammerhead-ribozyme). Preferably the nucleic acid should be able to bind to a target nucleic acid.

A functionally active nucleic acid means for example that if the nucleic acid e.g. comprises a protein encoding sequence the protein can be expressed (transcribed and/or translated) or e.g. if the nucleic acid comprises an oligonucleotide or a antisense RNA can bind to a target sequence.

The nucleic acid source may be any human and or non-human animal, plant, microorganism such as e.g. bacteria, fungi (e.g. yeast), virus, retrovirus.

In a special embodiment of the invention the nucleic acid contains a DNA sequence that encodes among others for the enzyme Chloramphenicol-Acetyl-Transferase (CAT). In another special embodiment of the invention the nucleic acid contains a DNA sequence that encodes beyond others for the HIV-1 protein tat.

Further, non-limiting examples of genes/cDNAs/coding sequences which could be (part of) the nucleic acid are immun-modulating genes such as those of cytokines like GM-CSF, IFN-γ, lL-2, alloantigen genes such as HLA-B7, "suicide" genes such as HSV-thymidine kinase, co-stimmulatory genes such as B7-1, tumor-associated antigenes, such as MART-1, tumor-suppressor genes such as p53, antisense genes that target oncogenes, antigrowth-factor genes, drug resistance genes.

The nucleic acid may comprise the sequences of one or more gene-regulatory sequences for the controlled expression, e.g. natural and/or synthetic promotors and/or enhancers. Promotors may be constiutive and/or inducible. The nucleic acid can comprise a basal promotor, a viral promotor, a promotor which is activated upon metabolic stimulation or upon treatment with a specific drug (e.g. tetracyclin), a cell-cycle specific promotor, a cell-type specific promotor and/or a chimeric promotor.

In a special embodiment of the invention the nucleic acid comprises the adenovirus major late promoter.

In another embodiment of the invention the nucleic acid comprises an inducible promoter which is activated upon stimulation with a specific trans-activating factor. In another special embodiment the inducible promotor is the HIV-1 long terminal repeat (LTR). This promotor is stimulated by the trans-activating factor tat which is specific for a particular cell type since its expression is restricted to HIV-infected cells.

In a special embodiment of the invention the transfection system may comprise different ratios of nucleic acid and histone. In a special embodiment of the invention the transfection system may comprise a nucleic acid to histone ratio of about 1 to 5, 1 to 4,5, 1 to 4, 1 to 3,5, 1 to 3, 1 to 2,5, 1 to 2, 1 to 1,5, 1 to 1 or less. In another special embodiment of the invention the transfection system may comprise a nucleic acid to histone ratio of about 1 to 5,5, 1 to 6, 1 to 6,5, 1 to 7, 1 to 8, 1 to 9, 1 to 10, 1 to 100 or more. In another special embodiment of the invention the nucleic acid to histone ratio may be 1,5 to 1, 2 to 1, 2,5 to 1, 3 to 1, 3,5 to 1, 4 to 1, 5 to 1, 10 to 1, 100 to 1 or more. Preferably the transfection system comprises a nucleic acid to histone ratio of 1 to 5. "Ratio" means the mass to mass ratio of histone protein and nucleic acid.

In a special embodiment of the invention the nucleic acid comprises one or more plasmids. These are for example the plasmids pCV1 (comprises tat gene and SV40 promotor) and pC15CAT (comprises HIV-1 LTR and CAT). The plasmids pCV1 (figure 1) and pC15CAT (figure 2) are described in Chandra et al. (1988) FEBS letters, Vol. 236, 282-286.

The transfection system may comprise additional compounds. These compounds are for example proteins, peptides, amino acids, chemical substances. Histones prepared from tissue and/or cells and/or cell nuclei may for example comprise additional compounds such as for example cofactors. If histones that have been isolated from tissue were used in the transfection system, the transfection system may comprise additional compounds assocciated with the histone protein and/or contained in the histone fraction such as for example in Johns fraction f3 and/or f2a.

The transfection system can be universally applied, e.g. whenever a nucleic acids should be brought into (the nuclei of) a cell or whenever a genetically engineered cell (recombinant cell, transgenic cell) should be provided.

Cells (e.g. target cells) are preferably any types of eukaryotic cells and/or cell lines, cells of tissues and/or tissue cultures and/or cells of animals and/or plants and or prokaryotic cells. Non-limiting examples are endothel cells, muscle cells, hamopoetic cells, steam cells, cells belonging to the immuno system (e.g. T-cells, B-cells, lymphoctes, macrophages), glia cells, tumor cells and/or derivatives thereof.

In a special embodiment of the invention the transfection system is used to bring a nucleic acid into the nuclei of a Jurkat cell.

In a special embodiment of the invention the transfection time (incubation time of transfection system with the cell) is about 10 minutes (min) or less, 30 min, 45 min, 60 min, 1,5 hours (hrs),2 hrs, 2,5 hrs, 3 hrs, 4 hrs, 5 hrs, 6 hrs, 7 hrs, 8 hrs, 9 hrs, 10 hrs, 11 hrs, 12 hrs, 14 hrs, 16 hrs, 18 hrs, 20 hrs, 24 hrs. The transfection time is the time the transfection system is incubated with a cell.

The invention also relates to the use of the new transfection system. The new transfection system can be used to transfer a nucleic acid into a cell.

The invention also relates to the use of a histone to transfer a nucleic acid into a cell. The invention also relates to the use of a histone protein in a method of making the transfection system. The invention also relates to the use of a histone protein in the transfection system.

The invention also relates to the use of a histone for making a transgenic cell.

The invention relates to the use of the transfection system for making a transgenic cell. A transgenic cell is a cell, into which a nucleic acid has been introduced.

The invention rates to a method of making a transgenic cell by using a histone. The invention further relates to a method of making a transgenic cell by using the transfection system.

A method of making a transgenic cell by using the transfection system is a two step process:
1. the transfection system is made prior to the transfection process by mixing and optionally incubating a histone and a nucleic acid and
2. the transfection system is than incubated with a cell, optionally for a particular time period (incubation time).

The method of making a transgenic cell by using the transfection system is characterized in that upon incubating the transfection system with a cell, the transfection system is transferred into the cell, e.g. into the nucleus of the cell.

The method of making the transgenic cell might be applied e.g. in gene therapy, in particular in somatic gene therapy.

The invention further relates to a transgenic cell made by transferring a nucleic acid into a cell by using a histone and/or the transfection system. Therefore, the transfection system can for example be used as tool in molecular biology.

The transfection system can for example be used in diagnosis and for the preparation of diagnostic tests. It can furthermore be used to prepare cells for the use in screening assays, e.g. for the screening of new protein and/or nucleic acid targets and/or the screening of highly specific new drugs.

The transfection system or a histone protein or a pharmaceutical composition thereof can be used for DNA-vaccination and/or for immune therapy.

The transfection system or histone protein can be used in gene therapy, in particular for somatic gene therapy and/or for the preparation a pharmaceutical composition that can be used in gene therapy, in particular for somatic gene therapy. A pharmaceutical composition should comprise an effective amount of the transfection system or histone protein. Such pharmaceutical composition can be used for ex vivo and/or in vivo application.
If the pharmaceutical composition comprises the transfection system, it may directly be used. If on the other hand, the pharmaceutical composition comprises only histone protein, the pharmaceutical composition must be mixed and optionally incubated with a nucleic acid prior to its used.

For ex vivo application for example hemapoetic stem cells (e.g. from bone marrow) can be transfected. Depending on the nucleic acid that is used transfected stem cells may for example be enabled to target specific cells, e.g. tumor cells and optionally deliver e.g. nucleic acids to those cells.

For in vivo application for example the use of a specific nucleic acid, e.g. with a promotor which is acivated only in a specific cell type and/or at a specific point of development and/or differentiation and/or cell cycle can ensure that the encoded protein is expressed in particular target cells, optionally at a special point of development/differentiation/cell cycle. For in vivo application the transfection system and/or a pharmaceutical composition thereof can for example be injected into particular tissue. This is possible because the transfection system is taken up by a cell very efficiently. This method may therefore be used for in vivo gene therapy, e.g. for gene therapy of cancer, in particular for solid tumors.

A pharmaceutical composition can be prepared in a manner known per se (e.g. Remingtons Pharmaceutical Sciences, Mack Publ. Co., Easton, PA), with pharmaceutically inert inorganic and/or organic excipients being used. Lactose, corn starch and/or derivatives thereof, talc, stearic acid and/or its salts, etc. can, for example, be used for preparing pills, tablets, coated tablets and hard gelatin capsules. Examples of excipients for soft gelatin capsules and/or suppositories are fats, waxes, semisolid and liquid polyols, natural and/or hardened oils, etc. Examples of suitable excipients for preparing solutions and/or syrups are water, sucrose, invert sugar, glucose, polyols, etc. Suitable excipients for preparing injection solutions are water, alcohols, glycerol, polyols, vegetable oils, etc. Suitable excipients for microcapsules, implants and/or rods are mixed polymers of glycolic acid and lactic acid.

In addition to the active ingredients and excipients, a pharmaceutical composition can also comprise additives, such as fillers, extenders, disintegrants, binders, lubricants, wetting agents, stabilizing agents, emulsifiers, preservatives, sweeteners, dyes, flavorings or aromatizing agents, thickeners, diluents or buffering substances, and, in addition, solvents and/or solubilizing agents and/or agents for achieving a slow release effect, and also salts for altering the osmotic pressure, coating agents and/or antioxidants.

A pharmaceutical composition may comprise the transfection system. The pharmaceutical composition may comprise histone H3 and/or histone H4. A pharmaceutical composition can be made by mixing the transfection system or a histone with the other components contained in the composition.

The dose of the pharmaceutical composition can vary within wide limits and is to be adjusted to the individual circumstances in each individual case.

The transfection system or the pharmaceutical composition may be an aerosol that can be inhaled, it may in a liquid that can be injected or drunken or it may be in a solid form for oral uptake.

The transfection system or the pharmaceutical composition can be used for the diagnosis, the prevention and/or the treatment of diseases, for example different kinds of cancer such as for example melanoma, carcinoma, lymphoma, basalioma, sarkoma, adenoma, neuroblastoma, glioma, teratoma, haemoblastoma in different kinds of tissues like for example head, brain, neck, skin, breast, stomache, kidney, bone, hepatic system, muscle, lung, prostate and different kinds of infections such as for example infections with different kinds of Influenza-virus, HIV (human immunodeficiency virus), HSV (herpes simplex virus), CMV (cytomegalo virus), HBV (hepatitis B-virus), HCV (hepatitis C-virus) and other types of hepatitis viruses, helicobacter pylori, malaria, cholera, typhus, grain and different kinds of genetic diseases such as for example cystic fibrosis, thalassaemia, cardiovascular diseases like atherosclerosis, retenosis and different kinds of immunological diseases such as for example autoimmuno diseases and different kinds of degenerative diseases such as for example Alzheimer's diseases, osteoporosis and diabetes, asthma, obesity.

### Detailed description of the invention

Some lipofection reagents have been studied in comparison to DEAE-dextran gene delivery system. DOTAP, Dosper and lipofectin have shown lower transfection efficiency. However, lipofectamine had a 2,5-fold better efficiency compared to that of DEAE-dextran. This could be due to it's stronger cationic character leading to a better condensation of DNA. This invention provides a novel and highly efficient DNA carrier system using the natural DNA-binding proteins, histones 3 and 4. Both proteins have nuclear location signal sequences which are responsible for their rapid accumulation in the cell nucleus. This transfection method (histonefection) is simple and not targeted to specific cell surface receptors. In transfection experiments the transactivation of HIV-1 LTR by the transactivator protein tat, both expressed in Jurkat cells was determined. HIV-1 LTR was hybridized to the gene of CAT which was used as reporter gene. In the experiments the level of expression of CAT was a direct measure of transactivation.

Compared to DEAE-dextran mediated transfection, histonefection resulted in a 7-fold increase of CAT expression. The maximum transfection efficiency of histones is dependent on concentration (DNA : histone ratio) and the incubation time. In a gel-retardation assay (gel shift assay) a maximum complex formation (between histone and DNA) was observed under the same conditions needed for the highest transfection efficiency. This ability of histones to increase the delivery and transgenic expression of foreign DNA in eukaryotic cells is not due to the positive ionic character of histone proteins since in experiments of the invention poly-lysine failed to catalyze transfection.
This invention provides a simple histone-mediated gene delivery system that can be used to translocate foreign genes into target cells.

The nuclear location and natural DNA-binding capability of histones make them ideal for delivering DNA into the cell nucleus. The simple construction of the histone-based carrier system means less variables need to be controlled, and there is a greater reliability in this method as compared with systems, which used receptor-mediated endocytose. In order to construct a natural ligand-polylysine complex with transferrin-receptor was used for transfection (Wagner et al., 1991). The renunciation of natural ligand to cell surface receptors by the use of unmodified histones in this method is potentially applicable to eukaryotic cells without specific surface receptors. Besides, the transfection with histones is more efficient than all the other tested transfection reagents. In comparison to transfection efficiency of DEAE-dextrane the histones mediates a 7-fold increase in transfection efficiency and only lipofectamine as one of tested lipofection reagents mediates a 2,5-fold increase.

In comparison to known methods and carrier systems the present invention provides a powerful transfection system which is very efficient, not time and material consuming, which is easy to perform and can be applied universally. The transfection system is not toxic for cells and/or animals and/or humans and/or plants since histones are cellular proteins. Due to the simple method of transfection the cells are not stressed by the procedure. Since histones shuttle the nucleic acids into nuclei of eukaryotic cells the majority of the nucleic acids that should be transported is delivered to the nucleus while cytoplasmatic enzyms have no or only little opportunity to destroy the transfection system. With this transfection system applied in gene therapy the immune responses e.g. of the human/animal should not be affected or damaged in comparison to viral vectors such as adeno-viral vectors.

Jurkat cells co-transfected with plasmids carrying that gene (HIV-1) and LTR sequence (HIV-1) hybridized to the reporter gene CAT are able to express CAT protein. The level of CAT expression is dependent on the transfection efficiency and the transgene expression of the tat protein. Different transfection systems were compared with respect to their transfection efficiency under optimal conditions of DNA : reagent ratio and the time of incubation.

Therefore, in a first step the different gene delivery systems (transfection systems) were compared at various DNA : reagent ratios. In table 2 the level of CAT expression at different DNA : reagent ratios employing various gene-delivery systems are shown. The transfection efficiency of DEAE-dextran under optimal conditions, standardized with each set of experiment, is shown for each experiment. The optimal concentration of these reagents with fixed amount of plasmid DNA that gave the best transfection efficiency was determined in each case. The varying amounts of plasmid DNA used in different lipofection experiments are used according to the instruction manuals supplied by the companies.

Lipofectin and Lipofectamine have the optimal effect at a DNA to transfection reagent ratio of 20:35, DOTAP (50:120) and Dosper (40:100) have similar requirements for their optimal activity, and histone 3 (40:200) and histone 4 (40:200) exhibit a close relationship in their DNA requirement for the maximum transgene expression. If the different transfection methods are compared at their optimal DNA : reagent ratio, the highest transgene expression is catalyzed by histonefection (transfection by histones). Except for Lipofectamine, the transfection efficiency of all the lipofection reagents was inferior to that of DEAE-Dextran.

In a second step the transfection period for optimal transgene expression was determined. The incubation period over which the DNA-reagent complex (transfection system) must be in contact with the cell to exert its optimal effect is dependent on several factors. The important ones are:
i) the chemical nature of the reagent (carrier);
ii) the cell type; and
iii) the concentration of DNA and the carrier.

The level of CAT expression at different periods of incubation for lipofectin, lipofectamin, DOTAP, Dosper, histone 3 and histone 4 was determined (table 3). In time-kinetic experiments, lipofectin, DOTAP and Dosper exhibit a similar behaviour with a transfection activity lower than that of DEAE-Dextran. The optimal period of incubation for lipofectin is between 3 to 5 h, whereas for DOTAP and Dosper between 7 and 9 hours. Lipofectamine which has a higher transfection potential than DEAE-Dextran, shows the highest gene expression in the first three hours; longer incubation periods with lipofectamine lead to a sharp decrease in the expression of CAT activity. This is due to the cytotoxicity of lipofectamine which was observed microscopically (data not shown). Time kinetic experiments with histone 3 and histone 4 gave a completely different pattern of activities at various incubation periods. Firstly, both the histones are active in promoting gene expression over a wide range of incubation period (1-24 h); secondly, both the histones exhibit their maximum effeciency in the same range (4-8 h); thirdly, the efficiency of both the histones in promoting gene expression is 7 to 9 times better than that of DEAE-Dextran. Therefore, histone 3 and histone 4 are non-toxic to cells and can be used over a wide range of incubation period as highly efficient gene carriers. This property may be important under in-vivo situation where contact period could be restricted by some factors.

Then, the effect of an anti-histone antibody on histone-mediated transfection on Jurkat cells was determined. The antibody is murine monoclonal antibody (IgG) which binds specifically to an antigen determinant, present on all five histone proteins (anti-histone, pan). The histone 3 and histone 4 lose their transfection potential by increasing amounts of antibodies. The inhibitory effect of histone IgG on histone-mediated CAT expression is concentration dependent. In these experiments, histones were preincubated with histone IgG and then mixed with DNA and added this transfection cocktail to the cells. These experiments reveal that the histone mediated gene transfection involves some specific structural requirements which are affected after its interaction with histone-lgG. Such an interaction may affect the histone-mediated DNA condensation, an important promoting factor in the gene delivery systems.

A gel-retardation assay performed with DNA-histone complexes that comprise varying amounts of histone and a constant concentration of DNA, showed that the migration of plasmid DNA alone was retarded gradually with increasing concentrations of histone H3. At the highest histone : DNA mass ratio (5:1) the gel mobility was completely retarded showing no migration of the complex. The effect of histone 4 on the mobility of plasmid DNA was shown by an analog experiment; the mass ratios between histone 4 and DNA were the same as for histone 3. The migration of plasmid DNA has the same retardation profile as for histone 3. Interestingly, the highest efficiency of both the histones in promoting transgene expression, the CAT expression, was at the same mass ratio (DNA : histone = 40:200) as observed in the gel retardation assay. The maximum retardation of mobility of histone-DNA complex for both the histones had a mass ratio between DNA and histone 1:5. It appears therefore, that DNA condensation is an important requirement for the transfection efficiency of histones.

Than, the effect of histone IgG on histone 3 and histone 4 mediated condensation of plasmid DNA was investigated in gel-retardation assay. The mass ratios between plasmid DNA and histone were same as in the above experiments without histone IgG. Varying amounts of histone were preincubated with histone IgG (10 µg/ml) and then added to the plasmid DNA. Contrary to what was expected, histone IgG intensifies the mobility retardation effect of both histones. In case of histone alone the total retardation of plasmid DNA mobility was observed at the mass ratio (DNA : histone; 1:5), whereas, in the presence of histone IgG a total retardation of DNA mobility at the mass ratio 1:3.3 (DNA : histone) was observed.

In a next step the effect of histones on the nucleolytic degradation of plasmid DNA was determined.

The protection of plasmid DNA against cellular nuclease is important for the functional activity of transgenic DNA, therefore the effect of DNase and restriction enzymes on the degradation of plasmid DNA alone and the DNA : histone complex (1:5) was studied. The probes were incubated with DNase alone, or in combination with restriction enzymes and separated on gels. The electrophoretic pattern of plasmid DNA pCV1 and pC15CAT DNase alone, or together with the restriction enzymes respectively. Lane 8 depicts the separation pattern of plasmid DNA treated with restriction enzymes only. The gel pattern of DNA treated with Dnase and restriction enzymes alone, or in combination are qualitatively the same. However, minor differences in the intensivity of some bands were seen. The effects of histone 3 and histone 4 on the nucleolytic degradation were very clear. At the DNA : histone ratio (1:5) which was used in the transfection experiments, the plasmid DNA is completely protected from nucleolytic degradation. This, probably, is due to histone-mediated condensation of DNA which is proven by the fact that DNA-histone complexes do not show any mobility on the gel, compared to the mobility of plasmid DNA without any treatment.
This feature of the transfection system is an important aspect for the functional activity of the transferred nucleic acid.

Therefore, the histone-based gene carrier system (transfection system) has several advantages:
i) the simple construction of this system needs less variables to be controlled; and
ii) there is a higher reliability in this method in comparison to systems, which use receptormediated endocytosis.

Histone 1 and histone H2A showed no transfection potential in this system when used as transfection reagent in the transfection system (data not shown). Polylysine (MW = appx. 15000) alone also had no effect in this system. The CAT expression in the presence of polylysine was 338 ± 42 pg/10 µg protein whereas, the control value without any addition, i.e. plasmid DNA alone, was 310 ± 51 pg/10 µg protein. Based on CAT expression, the transfection potential of histones 3 and 4 is approximately 200 times that of control value where plasmids were used alone.
- Figure 1:: Construction of the plasmid pCV1 with AdMLP (adenovirus major late promotor) and the cDNA of the tat gene.
- Figure 2:: Plasmid pC15CAT with SV40 promotor, HIV-1 LTR (C15) and CAT gene (CAT).
- Figure 3:: Comparison of different gene-delivery systems at various DNA:reagent ratios: a) Lipofectin; b) Lipofectamine; c) DOTAP; d) Dosper; e) histone 3 and f) histone 4.

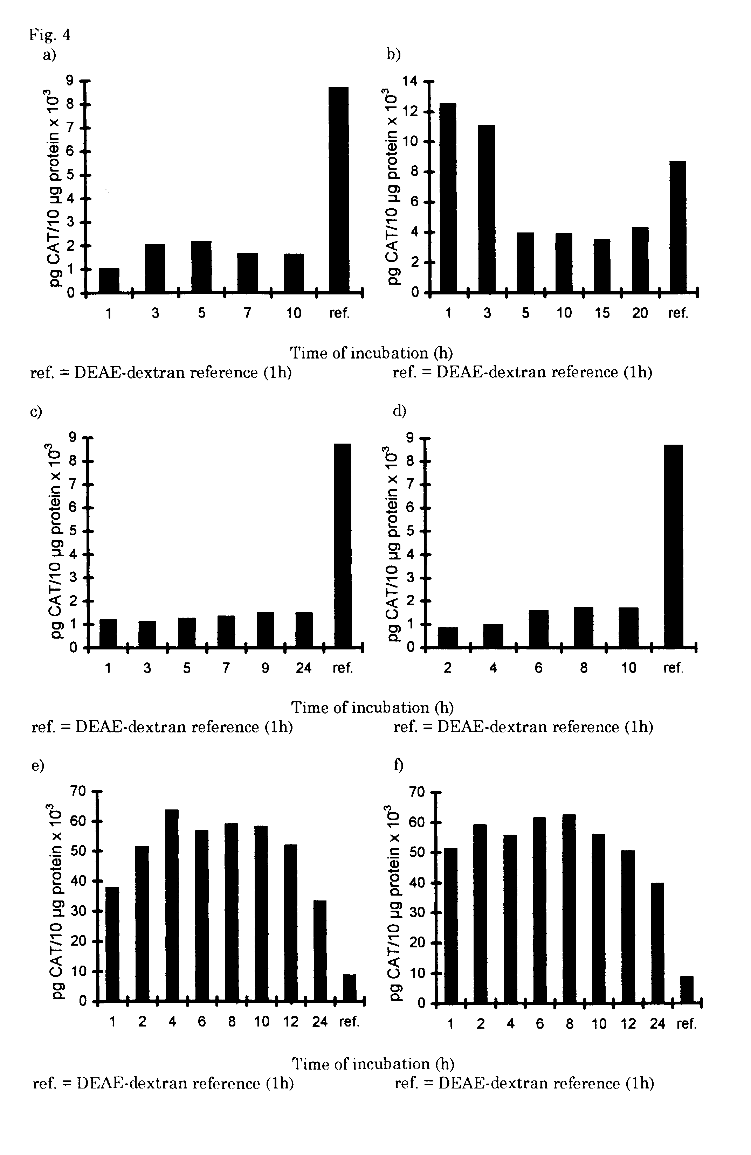

- Figure 4:: Transfection potential of different gene-delivery systems at various transfection periods: a) Lipofectin; b) Lipofectamine; c) DOTAP; d) Dosper; e) histone 3 and f) histone 4. The mass ratios for DNA:reagent complexes were based on the maximum activity for each reagent obtained in experiments shown in Fig. 3.

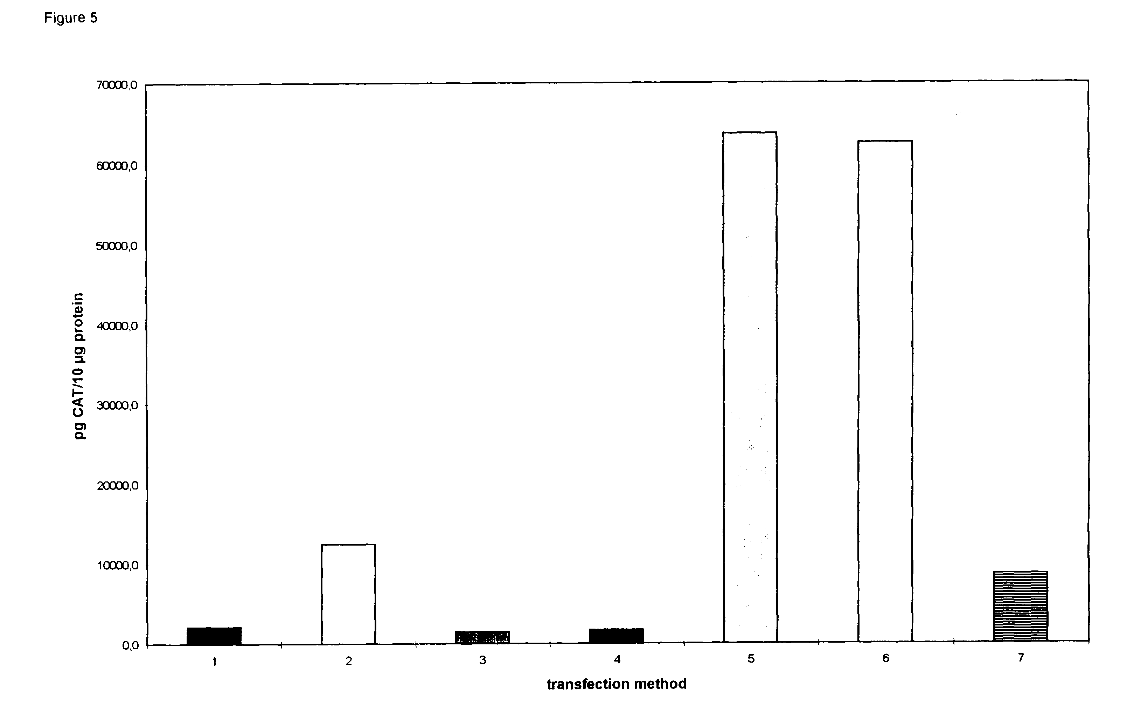

- Figure 5:: The diagram shows a comparison of the different transfection methods (1 to 7) with respect to the best incubation time for transfection. The best incubation time for each transfection method has been determined in separate experiments (results in table 3). For these experiments in each case the best ratio of DNA:reagent has been used. (1) lipofection (20 : 35; 5 h); (2) lipofectamine (20 : 35; 1 h), (3) DOTAP (50 : 120; 9 h), (4) Dosper (40 : 100; 8 h), (5) histone 3 (40 : 200; 4 h), (6) histone 4 (40 : 200; 8 h) and (7) DEAE-dextran (4 : 50, 1 h).

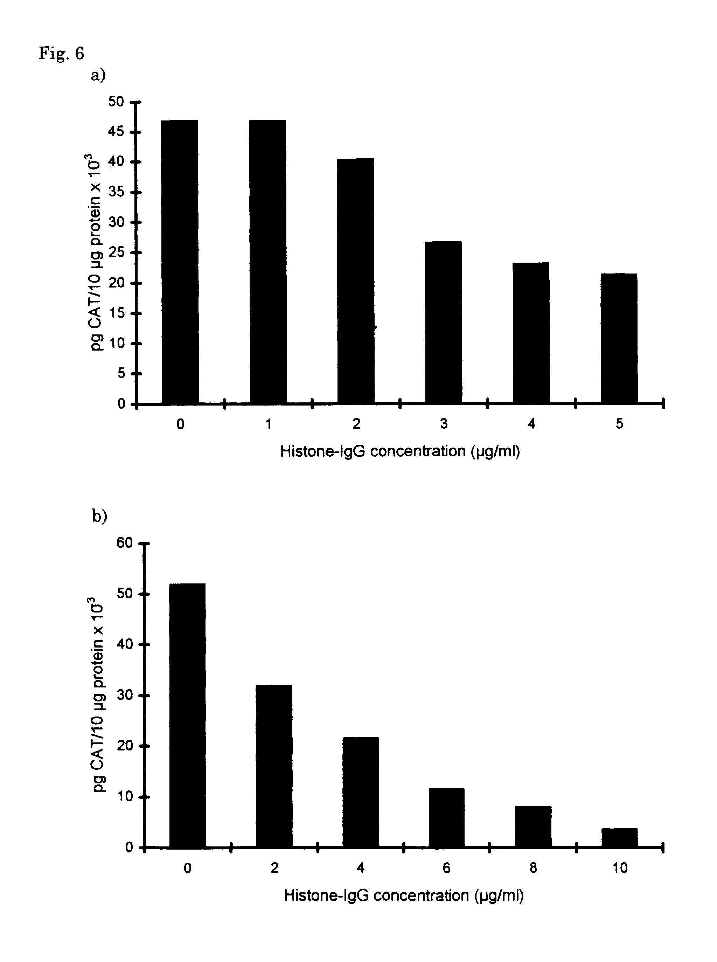

- Figure 6:: Effect of histone IgG on histone 3 (Fig. 3a) and histone 4 (Fig. 3b) mediated transgene expression in Jurkat cells.

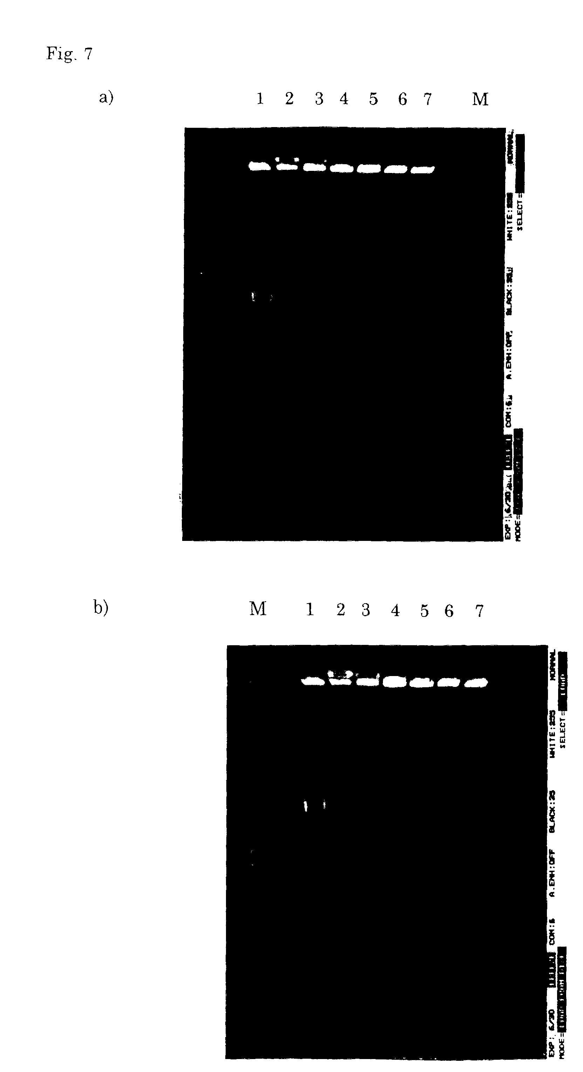

- Figure 7:: Electrophoretic mobility of plasmid DNA in the presence of various amounts of histone 3 (Fig. 4a) and histone 4 (Fig. 4b). Lane 1, plasmid DNA alone; Lane 2, (DNA:histone, 1:5); Lane 3, (DNA:histone, 1:3.3); Lane 4, (DNA:histone, 1:2.5); Lane 5, (DNA-histone, 1:2); Lane 6, (DNA:histone, 1:1.6); Lane 7, (DNA:histone, 1:1); M = 1 kb marker (Gibco BRL).

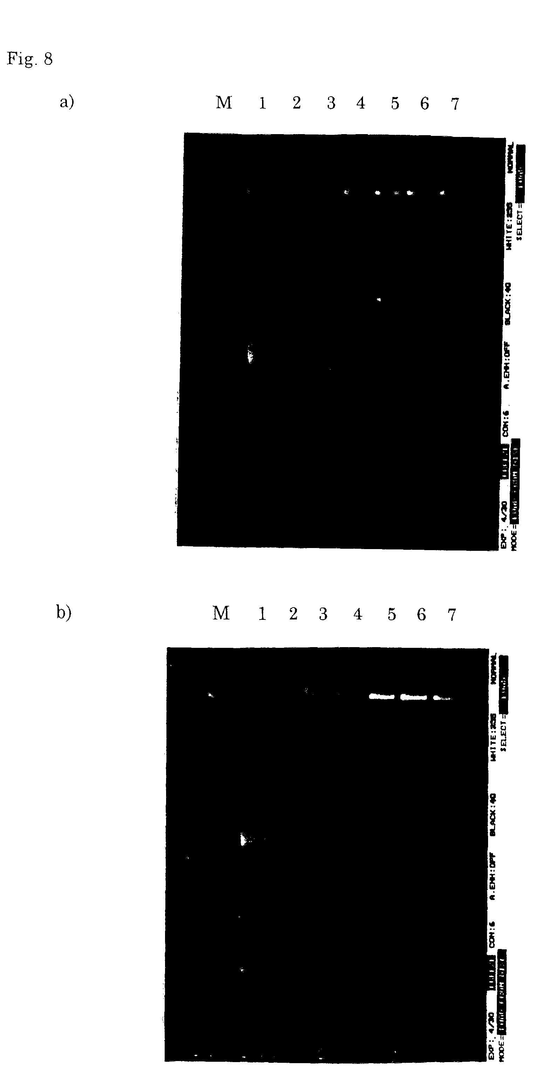

- Figure 8:: Electrophoretic mobility of histone-DNA complexes (histone 3, Fig. 5a; histone 4, Fig. 5b) in the presence of histone IgG (10 µg/ml). Lane 1, plasmid DNA alone; Lane 2, (DNA:histone, 1:5); Lane 3, (DNA:histone, 1:3.3); Lane 4, (DNA:histone, 1:2.5); Lane 5, (DNA:histone, 1:2); Lane 6, (DNA:histone, 1:1.6); Lane 7, (DNA:histone, 1:1); M = 1 kb marker (Gibco BRL).

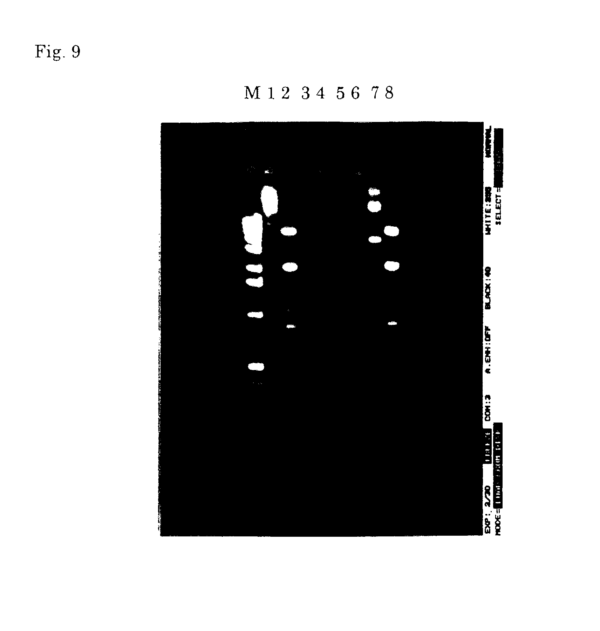

- Figure 9:: Effect of histones on the nucleolytic degradation of plasmid DNA measured by gel electrophoresis. Lane 1, plasmid DNA + DNase I; Lane 2, plasmid DNA + DNase I + restriction enzymes (see methods); Lane 3, DNA-histones 3 complex (DNA:histone 3, 1:5) + DNase I; Lane 4, DNA-histone 3 complex (DNA:histone 3, 1:5) + DNase I + restriction enzymes; Lane 5, DNA-histone 4 complex (DNA-histone 4, 1:5) + DNase I; Lane 6, DNA-histone 4 complex (DNA:histone 4, 1:5) + DNase I + restriction enzymes; Lane 7, plasmid DNA alone; Lane 8, plasmid DNA + restriction enzymes; M = 1 kb marker (Gibco BRL).

### EXAMPLES:

### Materials:

The culture medium (RPMI 1640 with 10% fetal calf serum and 1 % penicillin and streptomycin), the wash medium (RPMI) and fetal calf serum (FCS) were supplied by GIBCO BRL, Eggenstein. Jurkat cells (clone E 6-1, Weiss *et al.* 1985) an immortal cell line derived from T-lymphocytes were cultured in culture medium as described. ¹⁴C-acetyl-COA (4 mCi/mmol) was obtained from NEN/Dupont (Dreieich). All other reagents were analytic-grade of high purity obtained from Boehringer Mannheim, Mannheim (DOTAP and Dosper), FLUKA, Neu-Ulm (poly-lysine and histones 3 and 4), GIBCO BRL (lipofectin and lipofectamine) or from Sigma, Deisenhofen (DEAE-dextran).

### Plasmids:

The tat gene (clone 1) was cloned by way of cDNA-cloning, using poly (A)-selected RNA from HIV-1 (BH 10) infected cells (Arya *et al.,* 1985; Chandra *et al.,* 1988). The plasmid pCV1 (figure 1) was obtained by inserting viral cDNA containing the tat gene (clone 1) into the mammalian expression vector pCV which contains duplicated SV40 origins of replication (ori), adenovirus major late promotor, splice sites from adenovirus and mouse immunoglobulin genes, mouse dihydrofolate reductase cDNA, and SV40 polyadenylation signal. The HIV-1 LTR-CAT construct was obtained by inserting clone 15 DNA into pSV0-CAT at the Hind III site (Arya *et al.,* 1985; Arya *et al.,* 1986) and the resulting plasmid was termed pC15CAT (figure 2).

### Example 1: Comparable transfection experiments.

In transfection experiments 5 x 10⁶ Jurkat cells (with exception of DEAE-dextran experiment) per well were cultured in a 6-well tissue culture grade plate. Varying amounts between 5 and 25 µg per well of each plasmid DNA were used. To obtain varied concentrations of transfection reagents and varied ratio of DNA and reagent in a fixed final volume of transfection solution the volume of used buffer was altered. Results of these experiments are shown in table 2.
a) By using DEAE-dextran for transfection 2 x 10⁷ Jurkat cells were used. Jurkat cells were washed with serum-free medium and incubated at 37°C for 1 h in 2 ml serum-free medium containing 50 mM Tris-HCI (pH 7.3), DEAE-dextran (Mᵣ 500.000, 250 µg/ml) and 20 µg of each plasmid DNA, pCV 1 and pC15CAT. Then the cells were washed with RPMI medium (without serum) and incubated in 6 ml serum-containing medium at 37°C for 72 hrs.
b) For transfection with DOTAP (N-[1-(2,3-dioleoyloxy)-propyl]-N,N,N-trimethylammonium methylsulfate) or the following lipofection reagents the Jurkat cells were washed twice with serum-free medium and resuspended in 4,15 ml up to 4,65 ml culture medium. Simultaneously the plasmids (5 µg of each plasmid per 1 x 10⁶ cells) were diluted in 250 µl HEPES (20 mM). DOTAP was solved in concentrations between 5 - 30 µg/ml in 100 µl to 600 µl HEPES buffer and mixed gently with the plasmid solution.The mixture was incubated for 15 min. at room temperature. The DNA-DOTAP complex was added to the resuspended cells and incubated between 1 and 24 hrs. Then the medium was substituted with fresh RPMI medium and the cells were cultured for 72 hrs at 37°C and 5 % CO₂.
c) Dosper (1,3-dioleoyloxy-2-(6-carboxy-spermyl)-propylamind) in concentrations between 10-50 µg/ml and 4 µg per 1 x 10⁶ cells of each plasmids were diluted in HBS (20 mM HEPES, 150 mM NaCI) using volumina between 625 and 1000 µl, mixed and incubated for 15 min. at room temperature. After addition of the DNA-Dosper complex to the cells, which were diluted in 1,5 ml up to 1,85 ml serum-free medium, the cells were incubated for 2-10 hrs at 37°C. Then 0,5 ml FCS and 4,5 ml culture medium were added to the cells and the cells were incubated for 24 hrs with 5 % CO₂. Cells were centrifugated and resuspended in 5 ml fresh culture medium and then incubated for further 48 hrs under same conditions.
d) Lipofectin (a 1:1 [w/w] mixture of DOTAP (N-[1-(2,3-dioleoyloxy)-propyl]-N,N,N-trimethylammonium chloride (DOTMA) and dioleoyl phosphotidylethanolamine (DOPE)) or lipofectamine ( a 3:1 mixture of 2,3-dioleoyloxy-N-2-spermine carboxamidoethyl-N,N-dimethyl-1propanammoniumtriflouroacetat (DOSPA) and dioleoyl phosphotidylethanolamine) were used in concentrations between 5 and 45 µg/ml. They were diluted in 100 µl of 20 mM HEPES. 10 µg of pCV1 and pC15ACAT were diluted in 100 µl 20 mM HEPES. DNA and reagent were mixed and incubated for 15 min. at room temperature. The mixture was added to the cells, which were suspended in 0,8 ml serum-free medium. The cells were incubated for 1-10 hrs at 37°C. Then 4 ml RPMI medium were added and the cells were incubated for 72 hrs at 37°C and 5 % CO₂.
e) Cells, which should be transfected with histones, were resuspended in 1,05 ml up to 2,3 ml RPMI medium without serum and distributed in culture plates. Histones were diluted in 0 to 1,25 ml HBS. The plasmids (20 µg of each) were diluted in 200 µl of HBS and were added to the histone solutions. After incubation of the mixture for 20 min. the solutions of DNA-histone complexes (different ratio of DNA and histone in the solutions) were added to the cells with final concentrations of histones between 0 and 100 µg/ml. The transfection solutions were incubated between 0 and 24 hrs at 37°C and 5 % CO₂. Then 275 µl of FCS and 2,5 ml RPMI medium were added and the cells were cultered for 24 hrs at 37°C and 5 % CO₂. The cells were centrifugated and resuspended in 6 ml fresh culture medium and incubated for further 48 hrs at 37°C and 5 % CO₂.
f) Transfection experiments were performed with polylysine at the same conditions as with histones.

After transfection and incubation, cells were washed with phosphate buffered saline and suspended in 130 µl of 0.25 M Tris-HCI (pH 7.8). Cellular extracts were prepared by three cycles of freezing (in liquid nitrogen) and thawing (37°C). All cellular extracts were adjusted to equal protein concentration and heated for 15 min at 65°C to inactivate deacetylases in the extract.

### Example 2: Evaluation of transfection experiments.

### a) Protein quantification

The protein quantification of each cell lysate was carried out by Bradford method (Bradford, M.M. (1976) Anal. Biochem. 72, 248-254) method, which was performed in dublicate using 10 µl of cell lysate. Absorption readings (A₅₉₅) were converted to concentrations by comparison with a bovine serum albumin (Sigma) (20-100 µg/ml) standard curve after substraction of background values.

### b) CAT assay

For CAT assays, 30 µl of the cell extract were mixed with 20 µl of 100 mM Tris-HCI (pH 7.8) in a 6 ml glass scintillation vial. The vial was transferred to a water bath set to 37°C and 200 µl of freshly prepared CAT-reaction mixture (100 µl of 0.25 M Tris-HCI, pH 7.8; 50 µl of 5 mM chloramphenicol, 10 µl ¹⁴C- acetyl-CoA, 0.1 µCi; and 40 ml water) were added. To this mixture, 5 ml of a water-immiscible scintillation fluid (Econofluor, NEN/Dupont) was carefully overlaid and incubated at 37°C for 4 h. All transfections were performed minimum in dublicate for each set of experiments.

### Example 3: Determination of optimal histone to DNA ratio and optimal transfection rate by gel shift assay (gel retardation assay)

The optimal mass ratio of histone to DNA (histone:DNA) was detected in a gel retardation assay. The samples with a ratio from 1:1 up to 5:1 were electrophoresed on a 1 % agarose gel. In a gel retardation assay the migration of plasmid DNA is increasingly retarded as concentrations of the histones increased.

0,5 µg of each plasmid were mixed with 1 µg up to 5 µg of various histones in 20 µl of HBS, incubated for 20 min. at room temperature. After adding of 10 µl loading buffer (50 % 1xTE, 50 % glycerol and 0,05 % of brome phenolblue and xylene xyanol) and mixing the samples were electrophoresed on an 1 % agarose gele. The electrophoresed gele was stained with ethidium bromide and visualized with UV (λ = 254 nm). The visualized gel was scanned with a video camera by use of the Imager from Appligene (France) and was saved as tiff-file or printed by a thermo printer.
The optimal histone:DNA ratio that resulted in complete retardation (EMSA) was the same for both tested histones. With both histones DNA was completely retarded at a ratio of 5:1.

### Example 4: Determination of transfection rate using varying reagent to DNA ratios

Jurkat cells were transfected with histone 3/plasmids pCV1 and pC15CAT (1:1), histone 4/plasmids (pCV1 + pC15CAT, 1:1) and polylysine/plasmids at various protein:DNA ratios. For both histones, the optimal (histone 4) or nearly the optimal (histone 3) protein-DNA binding ratio also gave the best reporter gene expression in Jurkat cells. Experiments with polylysine leaded to no transfection in Jurkat cells.

According to standard protocols obtained from the companies, varying ratio of reagent to DNA was used to determine the best transfection efficiency for each protocol. In these experiments a fixed amount of plasmid DNA was used. Comparison of the results (table 2) demonstrates that lipofectamine gave 2,5 fold better transfection rate compared to DEAE-dextran. All other reagents leaded to a significant lower transfection efficiency. DEAE-dextran used as standard leads to 8718 pg CAT/10 µg protein (table 1).

### Example 5: Determination of the optimal incubation time

By using optimal reagent: DNA ratios for all transfection reagents the transfection efficiency at various incubation times was determined according to the procedure described in example 1 and 2. The results (table 3; table 1) indicated that lipofectamine and both histones leaded to better transfection efficiencies than DEAE-dextran. More, the results show that histones are threefold or more better than lipofectamin and nearly 7-fold better than transfection with DEAE-dextran. Transfection of Jurkat cells by using histones as carrier system lead to the best transfection efficiency.Transfection experiments with a mixture of both histones under same conditions as alone showed no no better transfection rate.

### Example 6: Inhibition of transfection (histonefection) by monoclonal anti-histone antibodies.

Inhibition of transfection by anti-histone antibodies proves that histones are responsible for the transfer of nucleic acids. Histones H3 and/or H4 in HBS were incubated with anti-histone antibodies (mouse-lgG1 against the pan-epitope, available from Boehringer Mannheim, No. 1 471, 519, Biochemicals Catalog 1995) (concentration of anti-histone antibodies 0-10 µg/ml) for 1 hour at 37°C. The rest of the procedure was performed as described in example 1 (incubation with plasmids, transfection) and example 3 (gel shift assay).

### Example 7: Preparation of histones from animal tissue (E.W. Johns (1964) Biochem. J. 92, 55-59).

Tissue from calf thymus is homogenized in 0.9 % NaCI-solution and centrifuged at 1100 g. The pellet is washed five times with 0.9 % NaCI. The pellet is homogenized with 5 % perchloric acid and zentrifuged at 1100 g two times. The pellet is suspended in ethanol and incubated at 4°C for 18 hours. The suspension is centrifuged (1100 g) and the pellet is washed with ethanol twice. The supernatants from ethanol extractions are collected, pooled and centrifuged at 2500 g. The supernatant is mixed with aceton (5 x volume) and then with concentrated HCI. The pellet is washed with aceton and dissolved in H₂O. Then ethanol (end concentration 80%) and HCI (end concentration 0.25 N) are added and the solution is dialysed 18 hours against ethanol. Then the solution is again dialysed under the same conditions for 4 hours. The pellet (f3) is washed once with ethanol and three times with aceton and dried in vakuum. The supernatant is mixed with aceton (3 x volume) and the pellet (f2a) is washed twice with aceton and dried in vakuum.

### Example 8: Plasmid degradation assay

0.5 µg of each plasmid was mixed with 5 µg of histones in 20 µl of HBS and preincubated for 20 min. at room temperatur. 10 units of DNase I and/or Pst I and Bam H1 were added and the restriction was performed for 1.5 h at 37°C. After addition of 10 µl loading buffer the probes were electrophoresed on a 1 % agarose gel and visualized with UV-light.

**Table 1:**

| In this table the optimized conditions and transfection efficiency of all transfection methods received from the mesure of CAT expression described above are summarized. | | | | |
|---|---|---|---|---|
| transfection reagent | ratio of DNA to transfection reagent | time of incubation [h] | amount of each plasmid [µg/10⁶ cells] | pg CAT/10 µg protein |
| DEAE-dextran (Sigma) | 4:50 | 1 | 1 | 8718 |
| lipofectin (GIBCO BRL) | 20:35 | 5 | 1 | 2161 |
| lipofectamine (GIBCO BRL) | 20:35 | 1 | 1 | 12500 |
| DOTAP (Boehringer Mannheim) | 50:120 | 9 | 5 | 1500 |
| Dosper (Boehringer Mannheim) | 40:100 | 8 | 4 | 1715 |
| histone 3 Johns Fraction III (Fluka) | 40:200 | 4 | 4 | 63689 |
| histone 4 (Fluka) | 40:200 | 8 | 4 | 62555 |
| poly-Lys (Fluka) | 40:0 - 40:250 | 6 | 4 | 0 |

**Table 2:**

| Determination of the best ration DNA : reagent In each case DEAE-dextran (ration 4 : 50) is used as standard. | |
|---|---|
| Lipofectine: | |
| ratio of DNA to lipofectin | pg CAT/10 µg protein |
| 4: 50 DEAE-dextran | 8717 |
| 20 : 0 | 470 |
| 20 : 5 | 3339 |
| 20 : 10 | 2760 |
| 20 : 15 | 3264 |
| 20 : 25 | 2955 |
| 20 : 35 | 4456 |
| 20 : 45 | 2082 |

| Lipofectamine: | |
|---|---|
| ratio of DNA to lipofectamine | pg CAT/10 µg protein |
| 4: 50 DEAE-dextran | 8717 |
| 20 : 0 | 615 |
| 20 : 5 | 1817 |
| 20 : 10 | 16102 |
| 20 : 15 | 17835 |
| 20 : 25 | 11831 |
| 20 : 35 | 20860 |
| 20 : 45 | 14176 |

| DOTAP: | |
|---|---|
| ratio of DNA to DOTAP | pg CAT/10 µg protein |
| 4: 50 DEAE-dextran | 8719 |
| 50 : 0 | 0 |
| 50 : 30 | 0 |
| 50 : 60 | 0 |
| 50 : 90 | 906 |
| 50 : 120 | 6381 |
| 50 : 180 | 5716 |

| DOSPER: | |
|---|---|
| ratio of DNA to Dosper | pg CAT/10 µg protein |
| 4 : 50 DEAE-dextran | 8719 |
| 40 : 0 | 0 |
| 40 : 25 | 0 |
| 40 : 50 | 0 |
| 40 : 75 | 245 |
| 40 : 100 | 4047 |
| 40 : 125 | 2751 |

| Histone 3: | |
|---|---|
| ratio of DNA to histone 3 | pg CAT/10 µg protein |
| 4 : 50 DEAE-dextran | 8717 |
| 40 : 0 | 0 |
| 40 : 25 | 0 |
| 40 : 50 | 0 |
| 40 : 75 | 0 |
| 40 : 100 | 0 |
| 40 : 125 | 12927 |
| 40 : 150 | 21845 |
| 40 : 175 | 28281 |
| 40 : 200 | 36208 |
| 40 : 225 | 7544 |
| 40 : 250 | 0 |

| Histone 4: | |
|---|---|
| ratio of DNA to histone 4 | pg CAT/10 µg protein |
| 4 : 50 DEAE-dextran | 8717 |
| 40 : 0 | 0 |
| 40 : 25 | 0 |
| 40 : 50 | 0 |
| 40 : 75 | 0 |
| 40 : 100 | 0 |
| 40 : 125 | 0 |
| 40 : 150 | 11212 |
| 40 : 175 | 28357 |
| 40 : 200 | 41116 |
| 40 : 225 | 12481 |
| 40 : 250 | 18607 |

**Table 3:**

| Determination of the optimal incubation time for transfection In each case DEAE-dextran (ration 4 : 50) is used as standard. | |
|---|---|
| Lipofectin: | |
| Incubation time [h] | pg CAT/10 µg protein |
| 4: 50 DEAE-dextran | 8718 |
| 1 | 1019 |
| 3 | 2037 |
| 5 | 2161 |
| 7 | 1653 |
| 10 | 1615 |

| Lipofectamine: | |
|---|---|
| Incubation time [h] | pg CAT/10 µg protein |
| 4: 50 DEAE-dextran | 8717 |
| 1 | 12500 |
| 3 | 11089 |
| 5 | 3955 |
| 10 | 3835 |
| 15 | 3533 |
| 20 | 4326 |

| DOTAP: | |
|---|---|
| Incubation time [h] | pg CAT/10 µg protein |
| 4 : 50 DEAE-dextran | 8718 |
| 1 | 1173 |
| 3 | 1108 |
| 5 | 1268 |
| 7 | 1348 |
| 9 | 1500 |
| 24 | 1506 |

| DOSPER: | |
|---|---|
| Incubation time [h] | pg CAT/10 µg protein |
| 4: 50 DEAE-dextran | 8718 |
| 2 | 850 |
| 4 | 988 |
| 6 | 1587 |
| 8 | 1715 |
| 10 | 1684 |

| Histone 3: | |
|---|---|
| Incubation time [h] | pg CAT/10 µg protein |
| 4: 50 DEAE-dextran | 8718 |
| 0 | 10256 |
| 1 | 37802 |
| 2 | 51523 |
| 4 | 63689 |
| 6 | 56849 |
| 8 | 59257 |
| 10 | 58332 |
| 12 | 52014 |
| 24 | 33332 |

| Histone 4: | |
|---|---|
| Incubation time [h] | pg CAT/10 µg protein |
| 4 : 50 DEAE-dextran | 8718 |
| 0 | 1544 |
| 1 | 51292 |
| 2 | 59139 |
| 4 | 55707 |
| 6 | 61614 |
| 8 | 62555 |
| 10 | 56092 |
| 12 | 50491 |
| 24 | 39710 |

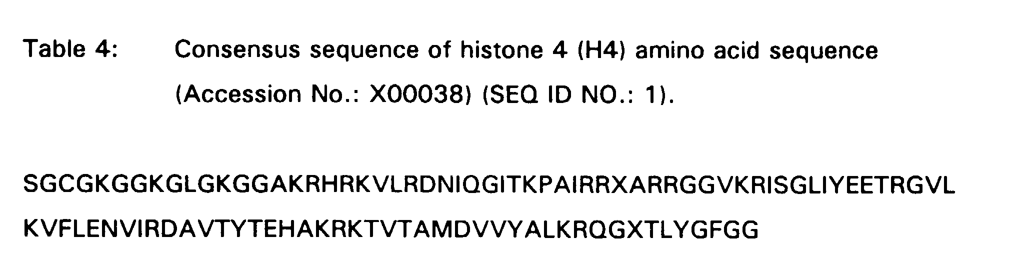

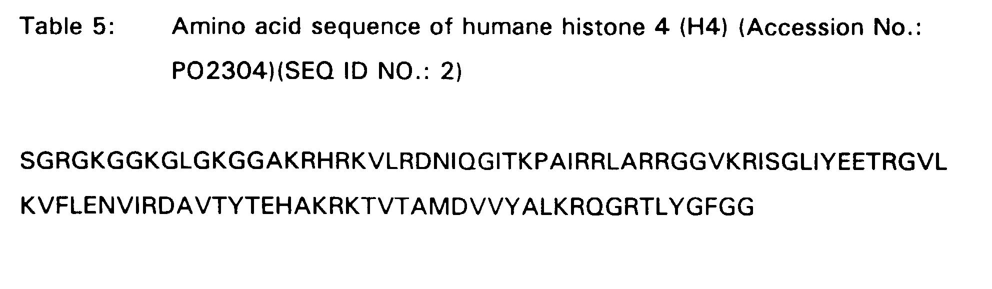

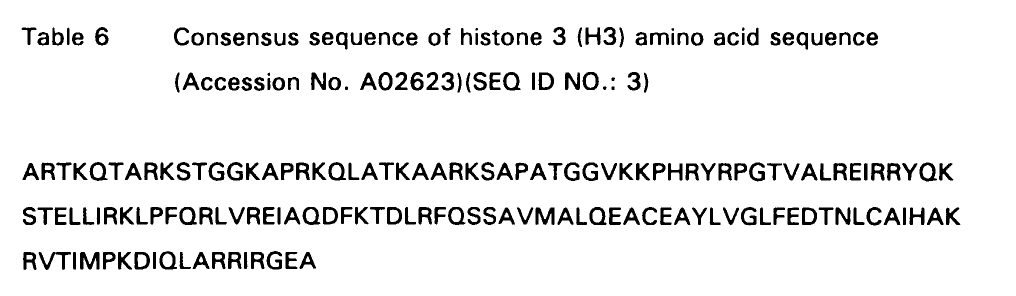

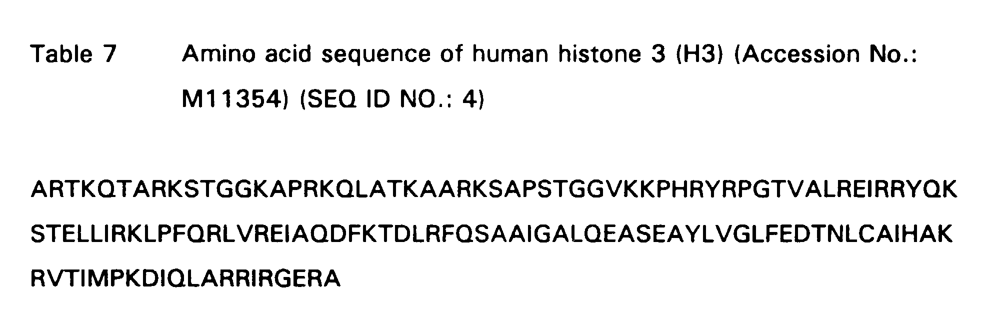

**Table 8**

| with CAT-Expression values for figure 6 | |
|---|---|
| Fig. 6a: Inhibition of histonefection with histone H3 by histone-lgG | |
| Histone-lgG concentration | pg CAT/10 µg protein |
| 0 | 45985 |
| 1 | 45980 |
| 2 | 39943 |
| 3 | 26382 |
| 4 | 22891 |
| 5 | 21146 |

| Fig. 6b: Inhibition of histonefection with histone H4 by histone-lgG | |
|---|---|
| Histone-lgG concentration | pg CAT/10 µg protein |
| 0 | 51830 |
| 2 | 31871 |
| 4 | 21496 |
| 6 | 11451 |
| 8 | 8011 |
| 10 | 3604 |

### References:

Arya, S. K. et al. Science 229, 69-73 (1985)
Arya, S. K. et al. Proc. Natl. Acad. Sci. U. S. A. 83, 2209-2213 (1986)
Bonner, W. M. J. Cell Biol. 64, 431-437 (1975)
Chandra A. , et al. FEBS Letters 236, 282-286 (1988)
Chen, C. A. et al BioTechniques 6, 632-638 (1988)
Chen, J. , et al. Human Gene Therapy 5, 429-435 (1994)
Choi, O.-R. B. et al. Cell 55, 17-26 (1988)
Cotten, M. et al. Proc. Natl. Acad. Sci. U. S. A. 87, 4033-4037 (1990)
Cotten, M. et al. J. Virolgy 67, 3777-3785 (1993)
Cotten, M. et al. Proc. Natl. Acad. Sci. U. S. A. 89, 6094-6098 (1992)
Curiel, D. T. et al. Amer. J. Resp. Cell & Mol. Biol. 6, 247-252 (1992)
Curiel, D. T. et al. Proc. Natl. Acad. Sci. U. S. A. 88, 8850-8854 (1991)
Demirhan, I. et al. Virus Genes 9, 113-119 (1994)
Dingwall, C. et al. EMBO J. 3, 1933-1937 (1984)
Felgner, P. L. et al. Proc. Natl. Acad. Sci. U. S. A. 84, 7413-7417 (1987)
Fraley, R. et al. Curr. Top. Microbiol.lmmunol. 96, 171-191 (1982)
Graessmann, M. et al. in "Microinjection and Organelle Transplantation
Techniques" (Celis, J. E. , Graessmann, A. , and Loyter, A. , eds.) pp. 3-13. Academia, London, 1986
Graham, F. L. et al. Virology 52, 456-467 (1973)
Holt, C. E. et al. Neuron 4, 203-214 (1990)
Kaneda, Y. et al. Science 243, 375-378 (1989)
Kawai, S. et al. Mol. Cell Biol. 4, 1172-1174 (1984)
Keown, W. A. et al. Methods Enzymol. 185, 527-537 (1990)
Loyter, A. et al. Proc. Natl. Acad. Sci. U. S. A. 79, 422-426 (1982)
Machy, P. et al. Proc. Natl. Acad. Sci. U. S. A. 85, 8027-8031 (1988)
Mannino, R. , J. et al. BioTechnique 6, 682-690 (1988)
Marceau, F. et al. Exper. Eye Res. 51, 645-650 (1990)
McCutchan, J. et al. J. Natl. Cancer Inst. 41, 351-357 (1968)
Moreland, R. B. et al. Mol. Cell. Biol. 7, 4048-4057 (1987)
Neumann, E. et al. EMBO J. 1, 841-845 (1982)
Nicolau, C. et al. Methods Enzymol. 152, 157-176 (1987)
Othani, K. et al. Nucleic Acids Res. 17, 1589-1604 (1989)
Plank, C. et al. J. Biol. Chem. 269, 12918-12924 (1994)
Wagner, E. et al. Proc. Natl. Acad. Sci. U. S. A. 88, 4255-4259 (1991)
Wagner, E. et al. Proc. Natl. Acad. Sci. U. S. A. 89, 7934-7938 (1992a)
Wagner, E. et al. Proc. Natl. Acad. Sci. U. S. A. 89, 6099-6103 (1992b)
Wagner, E. et al. Proc. Natl. Acad. Sci. U. S. A. 87, 3410-3414 (1990)
Wang, C.-Y. et al. Proc. Natl. Acad. Sci. U. S. A. 84, 7851-7855 (1987)
Weiss, R. A. et al. in RNA Tumor Virus, Cold Spring Harbor Lab., Vol. 1 and 2, New York, 1985
Wu, C. H. et al. J. Biol. Chem. 264, 16985-16987 (1989)
Wu, G. Y. et al. J. Biol. Chem. 266, 14338-14342 (1991)
Wu, G. Y. et al. J. Biol. Chem. 269, 11542-11546 (1994)
Zauner, W. et al. J. Virology 69, 1085-1092 (1995)
Zenke, M. et al. Proc. Natl. Acad. Sci. U. S. A. 87, 3655-3659 (1990)

## Claims

1. A transfection system that comprises histone or a derivative thereof and nucleic acid, wherein the histone is histone H3 or a derivative thereof or histone H4 or a derivative thereof.

2. A transfection system as claimed in claim 1, wherein the histone is acetylated.

3. A transfection system as claimed in one or more of claims 1 and 2, wherein the histone has been isolated from tissue.

4. A transfection system as claimed in one or more of claims 1 and 2, wherein the histone has been produced by recombinant expression of histone-encoding DNA.

5. A transfection system as claimed in one or more of claims 1 to 4, wherein the nucleic acid is a DNA.

6. A transfection system as claimed in one or more of claims 1 to 5, wherein the nucleic acid comprises a sequence that encodes for a protein.

7. A transfection system as claimed in one or more of claims 1 to 6, wherein the nucleic acid comprises a promotor for the controlled expression of the encoded protein.

8. A method of making a transfection system as claimed in one or more of claims 1 to 7 by mixing a histone with a nucleic acid.

9. A method of making a transgenic cell by incubating a transfection system as claimed in one or more of claims 1 to 7 with a cell.

10. The use of a transfection system as claimed in one or more of claims 1 to 7 for transferring a nucleic acid into a cell.

11. Use of a transfection system as claimed in one or more of claims 1 to 7 for the preparation of a pharmaceutical composition that can be used for preventing or treating a disease.

12. The use of a transfection system as claimed in one or more of claims 1 to 7 in gene therapy.

13. The use of transfection system as claimed in one or more of claims 1 to 7 for the preparation of a pharmaceutical composition that can be used in gene therapy.

14. The use of histone H3 and/or H4 for transferring a nucleic acid into a cell.

15. A pharmaceutical composition that comprised a transfection system as claimed in one or more of claims 1 to 7.

16. A pharmaceutical composition that comprises histone H3 and/or histone H4.


### Annex to the description

SEQUENCE LISTING

(1) GENERAL INFORMATION:

(i) APPLICANT:
(A) NAME: Hoechst Marion Roussel Deutschland GmbH
(B) STREET: -
(C) CITY: Frankfurt
(D) STATE: -
(E) COUNTRY: Germany
(F) POSTAL CODE (ZIP): 65926
(G) TELEPHONE: 069-305-7072
(H) TELEFAX: 069-35-7175
(I) TELEX: -

(ii) TITLE OF INVENTION: New transfection system for the transfer of
nucleic acids into encaryotic cells

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..102

(xi) SEQUENCE DESCRIPTION: SEQ ID NO: 1:

Ser Gly Cys Gly Lys Gly Gly Lys Gly Leu Gly Lys Gly Gly Ala Lys
1               5                   10                  15

Arg His Arg Lys Val Leu Arg Asp Asn Ile Gln Gly Ile Thr Lys Pro
            20                  25                  30

Ala Ile Arg Arg Xaa Ala Arg Arg Gly Gly Val Lys Arg Ile Ser Gly
        35                  40                  45

Leu Ile Tyr Glu Glu Thr Arg Gly Val Leu Lys Val Phe Leu Glu Asn
    50                  55                  60

Val Ile Arg Asp Ala Val Thr Tyr Thr Glu His Ala Lys Arg Lys Thr
65                  70                  75                  80

Val Thr Ala Met Asp Val Val Tyr Ala Leu Lys Arg Gln Gly Xaa Thr
                85                  90                  95

Leu Tyr Gly Phe Gly Gly
            100

(2) INFORMATION FOR SEQ ID NO: 2:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..102

(xi) SEQUENCE DESCRIPTION: SEQ ID NO: 2:

Ser Gly Arg Gly Lys Gly Gly Lys Gly Leu Gly Lys Gly Gly Ala Lys
1               5                   10                  15

Arg His Arg Lys Val Leu Arg Asp Asn Ile Gln Gly Ile Thr Lys Pro
            20                  25                  30

Ala Ile Arg Arg Leu Ala Arg Arg Gly Gly Val Lys Arg Ile Ser Gly
        35                  40                  45

Leu Ile Tyr Glu Glu Thr Arg Gly Val Leu Lys Val Phe Leu Glu Asn
    50                  55                  60

Val Ile Arg Asp Ala Val Thr Tyr Thr Glu His Ala Lys Arg Lys Thr
65                  70                  75                  80

Val Thr Ala Met Asp Val Val Tyr Ala Leu Lys Arg Gln Gly Arg Thr
                85                  90                  95

Leu Tyr Gly Phe Gly Gly
            100

(2) INFORMATION FOR SEQ ID NO: 3:

(i) SEQUENCE CHARACTERISTICS:
(A) LENGTH: 134 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..134

(xi) SEQUENCE DESCRIPTION: SEQ ID NO: 3:

Ala Arg Thr Lys Gln Thr Ala Arg Lys Ser Thr Gly Gly Lys Ala Pro
1               5                   10                  15

Arg Lys Gln Leu Ala Thr Lys Ala Ala Arg Lys Ser Ala Pro Ala Thr
            20                  25                  30

Gly Gly Val Lys Lys Pro His Arg Tyr Arg Pro Gly Thr Val Ala Leu
        35                  40                  45

Arg Glu Ile Arg Arg Tyr Gln Lys Ser Thr Glu Leu Leu Ile Arg Lys
    50                  55                  60

Leu Pro Phe Gln Arg Leu Val Arg Glu Ile Ala Gln Asp Phe Lys Thr
65                  70                  75                  80

Asp Leu Arg Phe Gln Ser Ser Ala Val Met Ala Leu Gln Glu Ala Cys
                85                  90                  95

Glu Ala Tyr Leu Val Gly Leu Phe Glu Asp Thr Asn Leu Cys Ala Ile
            100                 105                 110

His Ala Lys Arg Val Thr Ile Met Pro Lys Asp Ile Gln Leu Ala Arg
        115                 120                 125

Arg Ile Arg Gly Glu Ala
    130

(2) INFORMATION FOR SEQ ID NO: 4:

(i) SEQUENCE CHARACTERISTICS:
(A) LENGTH: 135 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..135

(xi) SEQUENCE DESCRIPTION: SEQ ID NO: 4:

Ala Arg Thr Lys Gln Thr Ala Arg Lys Ser Thr Gly Gly Lys Ala Pro
1               5                   10                  15

Arg Lys Gln Leu Ala Thr Lys Ala Ala Arg Lys Ser Ala Pro Ser Thr
            20                  25                  30

Gly Gly Val Lys Lys Pro His Arg Tyr Arg Pro Gly Thr Val Ala Leu
        35                  40                  45

Arg Glu Ile Arg Arg Tyr Gln Lys Ser Thr Glu Leu Leu Ile Arg Lys
    50                  55                  60

Leu Pro Phe Gln Arg Leu Val Arg Glu Ile Ala Gln Asp Phe Lys Thr
65                  70                  75                  80

Asp Leu Arg Phe Gln Ser Ala Ala Ile Gly Ala Leu Gln Glu Ala Ser
                85                  90                  95

Glu Ala Tyr Leu Val Gly Leu Phe Glu Asp Thr Asn Leu Cys Ala Ile
            100                 105                 110

His Ala Lys Arg Val Thr Ile Met Pro Lys Asp Ile Gln Leu Ala Arg
        115                 120                 125

Arg Ile Arg Gly Glu Arg Ala
    130                 135